# EUROPEAN PATENT APPLICATION

(11) **EP 4 737 901 A1**
(43) Date of publication of application: **06.05.2026**
(21) Application number: 24306819.4
(22) Date of filing: 29.10.2024
(51) Int. Cl.: G01N 33/58, B82Y 15/00

(54) **BIO-MARKED COMPOSITE PARTICLES**

(71) Applicant: Nexdot, 93230 Romainville (FR)
(72) Inventor: DUBERTRET, Benoit, 93230 Romainville (FR); CAO, Edgar, 93230 Romainville (FR); FLOCH, Camille, 93230 Romainville (FR)
(74) Representative: Icosa

(57) **Abstract**

The present invention relates to bio-marked composite particles.

## Description

### FIELD OF INVENTION

The present invention relates to bio-marked composite particles. More particularly, the present disclosure relates to bio-marked composite particles for detecting antibodies in a sample such as a patient's serum.

### BACKGROUND OF INVENTION

Some diseases may be diagnosed by analyzing the serum of a patient and checking if said serum includes specific antibodies associated with a particular disease. For instance, viral infections may be diagnosed through a blood test. However, each infection must be tested separately through a particular test. Accordingly, when a patient wishes to be tested for several infections, a lot of blood samples and large amounts of independent tests are necessary, which is costly and time consuming.

Accordingly, there is a need to improve the known process and to solve, at least partially, the said issues.

### SUMMARY

This invention relates to a bio-marked composite particle comprising
- a plurality of fluorescent nanoparticles encapsulated in a matrix comprising aluminium oxide, titanium oxide, zirconium oxide, hafnium oxide, silicon oxide or mixtures thereof; forming a composite particle and
- a plurality of biomolecules anchored at the surface of the matrix of the composite particle, said biomolecules comprising an anchor, the anchor comprising at least four histidine residues.

Such bio-marked composite particles emit, thanks to their fluorescent nanoparticles, at a particular wavelength when excited at an excitation wavelength. Thus, the presence of said bio-marked composite particles can be assessed with proper equipment. For instance, it is possible to identify said bio-marked composite particles in a flow cytometry process.

Moreover, antibodies may attach the biomolecules anchored to the composite particles. Further, fluorescent probes may attach to said antibodies, themselves attached to the composite particles. Fluorescent probes emit at a particular wavelength and their presence can be assessed with proper equipment. For instance, it is possible to identify fluorescent probes through a flow cytometry process.

Accordingly, the particle of the invention may interact with antibodies and fluorescent probes to form a couple that may be identified by two signals: the signal of the nanoparticles, and the signal of the fluorescent probe. Thus, when the two signals are present for a single particle, the formation of the couple is acknowledged.

Accordingly, by providing different bio-marked composite particles of the invention associating specific fluorescent nanoparticles to specific biomolecules in a single sample, it is possible to test the presence of a plurality of antibodies in said sample at the same time. In other words, each biomolecule would be associated to a single type of fluorescent nanoparticle that can be selectively identified.

Thus, when testing a sample comprising a plurality of antibodies, each antibody would recognize a specific biomolecule. As such, when the antibodies are probed by a fluorescent probe, a couple providing the signal of the specific fluorescent nanoparticles and the signal of the fluorescent probe is formed. Accordingly, considering that each couple of fluorescent nanoparticles/biomolecules are distinctively identified, acknowledging a couple is equivalent to acknowledging the presence of a specific antibody in the sample.

Therefore, the bio-marked composite particles of the invention allow testing multiple antibodies in a single test, which is more time efficient, and simpler.

Moreover, it is noted that providing an anchor comprising at least four histidine residues enhances the strength of the bounding between the composite particle and the biomolecule. For instance, such an anchor allows biomolecules to remain bound to the composite particle even after at least five washes.

In some embodiments, the composition of the matrix is AlₓZr_{y}O (I) ; wherein x, and y respectively represent the stoichiometric ratios of Aluminum and Zirconium; x is in the range from 0 to 2/3 y is in the range from 0 to 1/2; and (3/2x+2y)=1.

Such matrixes are stable and especially resistant to heat and to humidity. Moreover, it has been surprisingly found that embedding fluorescent nanoparticles in a matrix comprising a mixture of alumina and zirconia is especially efficient to prevent fluorescent nanoparticles degradation. Also, such particles are able to disperse in a water-based dispersion. Thus, they are durable even in contact with biological solutions such as serum or urine.

In some embodiments, the composition of the matrix is pure ZrO₂.

Zirconia matrix has shown better binding with the biomolecules comprising anchors comprising histidine residues, compared to other matrixes. Thus, zirconia matrix allows providing more stable bio-marked particles.

In some embodiments, the matrix is a metal oxide compound of formula AlₓZr_{y}M_{z}O, wherein x is in the range from 0 to 2/3, y is in the range from 0 to 1/2, M represents one or more metal elements selected from the group of Si, Ti, Hf, Ge, Sn, or mixtures thereof, and z is in the range from 0 to 2/5, 0 excluded.

In some embodiments, the anchor comprises at least four consecutive histidine residues.

Such anchors provide stronger bounding to the composite particle.

In some embodiments, the matrix is obtained by nebulization of a composition in a heated enclosure, said composition comprising
- fluorescent nanoparticles; and
- precursors of aluminium oxide, titanium oxide, zirconium oxide, hafnium oxide, silicon oxide or mixtures thereof.

Nebulization is a reliable and effective method to obtain composite particles suitable for the method of the invention.

In some embodiments, the precursors of aluminium oxide, titanium oxide, zirconium oxide, hafnium oxide or silicon oxide are selected from the group of carboxylates, carbonates, thiolates, alkoxides, oxides, sulfates, phosphates, nitrates, acetates, chlorides, bromides, acetylacetonate or a mixture thereof; preferably the precursors are alkoxides.

In some embodiments, the fluorescent nanoparticles are quantum dots.

In some embodiments, the fluorescent nanoparticles are quantum nanoplates.

Such fluorescent nanoparticles emission is narrower than other nanoparticles, which is particularly convenient for providing couples of fluorescent nanoparticles/biomolecules that can be independently identified. Moreover, these fluorescent nanoparticles are stable and provide a good quantum yield.

In some embodiments, the biomolecules are selected from the group consisting of proteins, nucleic acids, hormones, antigens, antibody, viruses' parts or a mix thereof.

These types of biomolecules are particularly relevant for medical applications. Viruses are especially pertinent for virology tests.

The present invention also concerns a method for synthetizing a bio-marked composite particle comprising:
- Providing composite particles comprising a plurality of fluorescent nanoparticles encapsulated in a matrix comprising aluminium oxide, titanium oxide, zirconium oxide, hafnium oxide, silicon oxide or mixtures thereof;
- Providing biomolecules comprising an anchor, the anchor comprising at least four histidine residues;
- Mixing the composite particles and the biomolecules in a buffer.

This method allows synthetizing particles that present the abovementioned advantages, allowing to perform multiple tests in a single sample.

In some embodiments, prior to providing biomolecules comprising an anchor, the method comprises providing biomolecules and grafting an anchor to said biomolecules, the anchor comprising at least four histidine residues, preferably four consecutive histidine residues.

Some biomolecules naturally present at least four terminal histidine residues. However, some biomolecules do not present this property. Accordingly, grafting histidine residues to a biomolecule allows guarantying that said biomolecule comprises an anchor with at least four histidine residues. Thus, said biomolecule benefits from the advantages of having such an anchor, and may be treated more easily by the method of the invention.

The present invention also concerns a method for detecting antibodies for a biomolecule in a sample, the method comprising:
- Sampling a fluid sample of a patient;
- Adding the sample to a dispersion comprising the bio-marked composite particles of any of the precedent aspects comprising said biomolecule, to obtain a test sample;
- Adding a fluorescent probe to the test sample to obtain a probed test sample;
- Analyzing the probed test sample through a flow cytometry process.

The fluid sample may be blood, urine, serum or any fluid sample that may comprise biomolecules that should be tested.

The method for detecting antibodies provides the above technical advantages. In particular, the method of the invention allows testing multiple antibodies in a single test, which is more time efficient, and simpler.

### DEFINITIONS

In the present invention, the following terms have the following meanings:

"at%" refers to the atomic percentage of an element in a compound. In this disclosure, the atomic percentage for a metallic element of a metal oxide matrix is given without considering oxygen. For instance, Al₂O₃ contains 0 at% of aluminum as no other metallic element is present in the metal oxide.

"wt%" refers to the weight percentage of a compound in a formulation or of an element in a compound.

"Core/shell" refers to heterogeneous nanostructure comprising an inner part: the core, overcoated on its surface, totally or partially, by a film or a layer of at least one atom thick material different from the core: the shell. Core/shell structures are noted as follows: core material/shell material. For instance, a particle comprising a core of CdSe and a shell of ZnS is noted CdSe/ZnS. By extension, core/shell/shell structures are defined as core/first-shell structures overcoated on their surface, totally or partially, by a film or a layer of at least one atom thick material different from the core and/or from the first shell: the second-shell. For instance, a particle comprising a core of CdSe, a first-shell of CdS and a second-shell of ZnS is noted CdSe/CdS/ZnS. Core/shell nanostructure also include nanoparticles in which the central part: the core, is embedded - or encapsulated - by a layer of material disposed on the core: the shell, said shell having a gradient of composition from the core to the outside of the shell. In this case, the composition of the nanoparticle is changing smoothly - for instance continuously - from the core composition - for instance CdSe - to the outside composition of the shell - ZnS for instance. There is no precise boundary between core and shell but properties in centre of the core are different from properties on the outer boundary of shell. In addition, core and shell may have different shapes, for instance a dot - a nanosphere or a nanocube or any other nanocluster - is provided as a core and shell is grown laterally around the core, yielding an heterostructure with shape of a nanoplate but comprising a dot inside the nanoplate.

**"Doped"** refers to a composition of material with a crystalline structure - core or shell - in which a dopant element is substituted to the main element. Such compositions are noted QD:Dopant in the following. For instance, a ZnSe quantum dot may be doped with Manganese (Mn) so that a part of Zn elements are substituted by Mn elements, and noted ZnSe:Mn.

**"Encapsulated"** refers to a state in which a material - or a matrix - coats, surrounds, embeds, contains, comprises, wraps, packs, or encloses a plurality of particles, which may be nanoparticles or composite particles.

**"Fluorescent"** refers to the property of a material that emits light after being excited by absorption of light. Actually, light absorption drives said material in an energetically excited state, which eventually relaxes by emission of light of lower energy, *i.e.,* of longer wavelength - red shifted.

**"Loading charge"** refers to the mass ratio between the mass of particles comprised in a formulation and the mass of said formulation. For the sake of clarity, 10 g of particles mixed with 90g of a matrix defines a loading charge of 10%.

**"Mean size"** refers to a size of a population of particles, obtained by a mathematical mean of sizes of each individual particle of the population. Practically, the mean size may be determined by electronic microscopy: size of each particle visible in the microscopy is evaluated by fitting each particle with a circle whose diameter defines the size of the particle, then computing the mean of all individual sizes to obtain the mean size. Other methods, such as light scattering may be used to determine indirectly the mean size of the population of particles. Experimentally, particles are always obtained in the form of population of particles. By extension in this disclosure, the mean size of a particle is the mean size of the population of particles which has been synthesized. For the sake of clarity, a particle having a mean size between 0 nm and 250 nm is a particle representative of a population of particles having a mean size between 0 nm and 250 nm.

**"Monodisperse"** refers to a population of particles, wherein the distribution of size populations has a PolyDispersity Index (PDI) lower than 0.3, preferably lower than 0.2.

**"Nanometric size"** refers to a size of matter where at least one physical property is directly governed by size. For semi-conductive nanoparticles, nanometric size has to be defined with the average Bohr radius of an electron/hole pair in the material in which quantum effects appear due to confinement. For semi-conductive materials disclosed here, quantum effects appear for size in at least one dimension of the object below 20 nm, preferably below nm, more preferably below 5 nm. For conductive particles, nanometric size has to be defined from the resonance of oscillations of free electron gas density, which becomes relevant for optical measurements in the range from 380 nm to 3 µm for size in at least one dimension of the object below 20 nm for conductive materials disclosed here.

**"Nanoparticle"** refers to a particle having a size in at least one of its dimensions below 100 nm. For a nanosphere, diameter should be below 100 nm. For a nanoplate, thickness should be below 100 nm. For a nanorod, diameter should be below 100 nm.

**"Nanoplate"** refers to a nanoparticle having a 2D-shape, *i.e.,* having one dimension smaller than the two others; said smaller dimension is below 100 nanometers. In the sense of the present invention, the smallest dimension (hereafter referred to as the thickness) is smaller than the other two dimensions (hereafter referred to as the length and the width) by a factor (aspect ratio) of at least 1.5.

**"PLQY"** refers to photoluminescence quantum yield, *i.e.,* the ratio between the number of fluorescence photons and the exciting photons

**"Range"** refers to a range of values. Lower limit and upper limit of the range are included in the range.

**"Semi-conductive nanoparticles"** refers to particles made of a material having an electronic structure corresponding to semi-conductive materials known in electronic industry but having a **nanometric size.** Due to their specific electronic structure, semi-conductive materials behave as high-pass absorbing materials. Indeed, light having a wavelength more energetic than band gap may be absorbed by the semi-conductive material, yielding an electron/hole pair, an exciton, which later recombine in the material and dissipate heat, or emit light, or both. On the contrary, light having a wavelength less energetic than band gap cannot be absorbed: semi-conductive material is transparent for these wavelengths. In macroscopic semi-conductive materials, visible light is generally absorbed while near/mid infra-red light is not absorbed. When semi-conductive particles have a **nanometric size,** confinement - *i.e.,* shape and nanometric size - governs electronic structure following the rules of quantum mechanics and light absorption may be limited to UV range or UV and high energy visible light.

### BRIEF DESCRIPTION OF THE DRAWINGS

**Figure 1A-1F** illustrates various nanoparticles (1) with homostructure (A) or heterostructures: spherical core/shell (B), spherical core/shell/shell (C), dot in plate (D), nanoplate core/shell (E) and nanoplate core/crown (F).
**Figure 2** is a schema illustrating a bio-marked composite particle according to the invention.
**Figure 3** is a schema illustrating a bio-marked composite particle according to the invention attached to an antibody and a fluorescent probe.
**Figure 4** is a graphic representing the intensity of fluorescence for a population of bio-marked composite particles. X axis represents the fluorescence intensity at the emission wavelength of the fluorescent nanoparticles while Y axis represents the fluorescence intensity at the emission wavelength of the fluorescent probe. The test is a flow cytometry test, for a sample comprising Sars-Cov2 bio-marked composite particles and a control sample.
**Figure 5** is a graphic representing the intensity of fluorescence for a population of bio-marked composite particles. X axis represents the fluorescence intensity at the emission wavelength of the fluorescent nanoparticles while Y axis represents the fluorescence intensity at the emission wavelength of the fluorescent probe. The test is a flow cytometry test, for a sample comprising Neisseria meningitidis bio-marked composite particles and a control sample.
**Figure 6** represents a histogram representing, for a population of bio-marked composite particles, the mean fluorescence intensity at the emission wavelength of the fluorescent probe functions of the number of consecutive histidine residues included in the anchor of the biomolecule of the bio-marked composite particles.

### DETAILED DESCRIPTION

The following description provides examples for achieving the invention. It shall be read as descriptive rather than limitative.

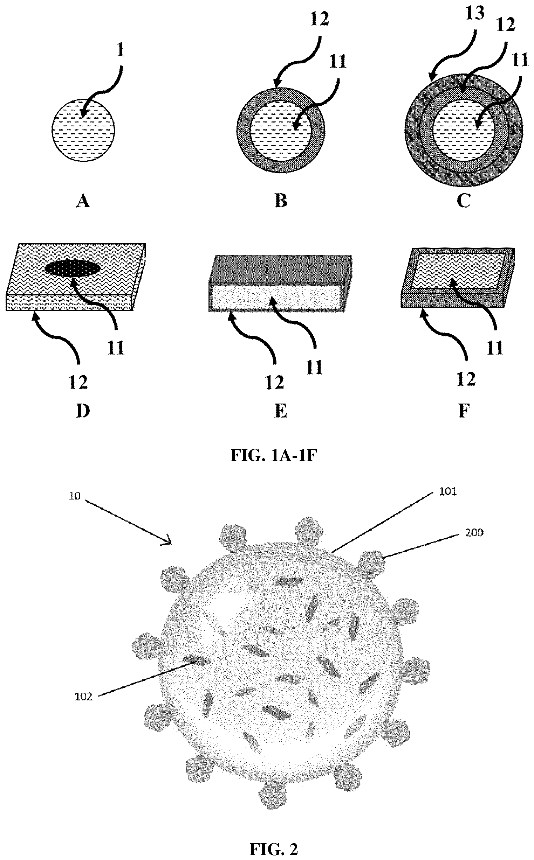

Figure 2 is a schema illustrating a bio-marked composite particle 10 according to the invention. The bio-marked composite particle 10 comprises a plurality of fluorescent nanoparticles 102, more generally semi-conductive nanoparticles, encapsulated in a matrix 101. A plurality of biomolecules 200 are anchored at the surface of the matrix.

### Semi-conductive nanoparticles

In this disclosure, the semi-conductive nanoparticles can be any kind of semi-conductive material prepared in the form of nanoparticles.

Suitable semi-conductive nanoparticles may be selected from fluorescent semi-conductive nanoparticles known as quantum dots, which may have various composition, shape and structure.

### Quantum dots composition

In one embodiment, the quantum dots comprise a material of formula

MₓQ_{y}E_{z}A_{w} (II)

in which M is selected from the group consisting of Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb, Cs or a mixture thereof; Q is selected from the group consisting of Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb, Cs or a mixture thereof; E is selected from the group consisting of O, S, Se, Te, C, N, P, As, Sb, F, Cl, Br, I, or a mixture thereof; and A is selected from the group consisting of O, S, Se, Te, C, N, P, As, Sb, F, Cl, Br, I, or a mixture thereof. x, y, z and w are independently a decimal number from 0 to 5; x, y, z and w are not simultaneously equal to 0; x and y are not simultaneously equal to 0; z and w are not simultaneously equal to 0.

Especially, quantum dots may comprise a material of formula MₓE_{y}, in which M is Zn, Cd, Hg, Cu, Ag, Al, Ga, In, Si, Ge, Pb, Sb or a mixture thereof; and E is O, S, Se, Te, N, P, As or a mixture thereof. x and y are independently a decimal number from 0 to 5, with the proviso that x and y are not 0 at the same time.

In a specific embodiment, quantum dots comprise a material selected from the group consisting of CdS, CdSe, CdTe, ZnS, ZnSe, ZnTe, HgS, HgSe, HgTe, HgO, GeS, GeSe, GeTe, SnS, SnSe, SnTe, PbS, PbSe, PbTe, GeS₂, GeSe₂, SnS₂, SnSe₂, CuInS₂, CuInSe₂, CuInZnS, CuInZnSe, AgInS₂, AgInSe₂, CuS, Cu₂S, Ag₂S, Ag₂Se, Ag₂Te, FeS, FeS₂, InP, Cd₃P₂, Zn₃P₂, CdO, ZnO, FeO, Fe₂O₃, Fe₃O4, Al₂O₃, TiO₂, MgO, MgS, MgSe, MgTe, AlN, AlP, AlAs, AlSb, GaN, GaP, GaAs, GaSb, InN, InP, InAs, InSb, InAsP, TlN, TlP, TlAs, TlSb, MoS₂, PdS, Pd4S, WS₂, CsPbCl₃, PbBr₃, CsPbBr₃, CH₃NH₃PbI₃, CH₃NH₃PbCl₃, CH₃NH₃PbBr₃, CsPbI₃, FAPbBr₃, FAPbI₃ (where FA stands for formamidinium), or a mixture thereof.

In one embodiment, quantum dots are doped with at least one transition metal such as, for example, Sc, Y, Ti, Zr, Hf, Rf, V, Nb, Ta, Dd, Cr, Mo, W, Sg, Mn, Tc, Re, Bh, Fe, Ru, Os, Hs, Co, Rh, Ir, Ni, Pd, Pt, Cu, Ag or Au. Preferably, quantum dots are doped with Mg, Mn, Al, Cu, Te or Ag. In particular, ZnSe quantum dots doped with Mn :ZnSe:Mn, ZnSe quantum dots doped with Al: ZnSe:Al, or ZnSe quantum dots doped with Mg: ZnSe:Mg are suitable. Amount of dopant is preferably less than 10 wt%. Dopant amount of 5 wt% or 2 wt% are suitable.

### Quantum dots shape

In one embodiment, quantum dots may have different shapes, provided that they present a nanometric size leading to quantum confinement in the nanoparticle.

Quantum dots may have nanometric sizes in three dimensions, allowing quantum confinement in all three spatial dimensions. Such quantum dots are for instance nanocubes or nanospheres.

Quantum dots may have a nanometric sizes in two dimensions, the third dimension being larger: quantum confinement is in two spatial dimensions. Such quantum dots are for instance nanorods, nanowires or nanorings.

Quantum dots may have a nanometric size in one dimension, the other dimensions being larger: quantum confinement is in one spatial dimension only. Such quantum dots are for instance nanoplates, nanosheets, nanoribbons or nanodisks. Nanoplates are especially interesting in this disclosure because absorption cross section *- i.e.,* efficiency to capture a photon of incident light on the quantum dot - is ten times higher than a nanosphere having the same composition and structure emitting at the same wavelength. This higher cross section improves significantly sensitivity of assays. In addition, nanoplates have narrower FWHM emission spectra - typically below 40 nm - and shorter photoluminescence decay time - by one order of magnitude - as compared to spherical quantum dots.

The exact shape of quantum dots defines confinement properties; then electronic and optical properties.

### Quantum dots structure

In an embodiment, quantum dots are homostructures. By homostructure, it is meant that the quantum dot is homogenous and has the same local composition in all its volume. A homogeneous spherical quantum dot 1 is illustrated in figure 1A.

In an alternative embodiment, quantum dots are heterostructures. By heterostructure, it is meant that the quantum dot is comprised of several sub-volumes, each sub-volume having a different composition from neighbouring sub-volumes. In a particular embodiment, all sub-volumes have a composition defined by formula (II) disclosed above, with different parameters, *i.e.,* elemental composition and stoichiometry.

Example of heterostructure are core/shell nanoparticles, the core (11) having any shape disclosed above. A shell (12) is a layer covering totally or partially the core. A particular example of core/shell heterostructure is a multi-layered structure comprising a core (11) and several successive shells (12, 13). For convenience, these multi-layered heterostructures are named core/shell hereafter. Core (11) and shell (12,13) may have the same shape - sphere in sphere for example - or not - sphere in plate for instance. A core/shell spherical nanoparticle is illustrated in figure 1B. A core/shell/shell spherical nanoparticle is illustrated in figure 1C. A sphere in plate nanoparticle is illustrated in figure 1D - also named a dot in plate. A core/shell nanoplate is illustrated in figure 1E.

Another example of heterostructure are core/crown nanoparticles, the core having any shape disclosed above. A crown is a band of material disposed on the periphery of the core. This heterostructure is particularly useful with cores being nanoplates and crown disposed on the edges of the nanoplate. A core/crown nanoplate is illustrated in figure 1F.

These heterostructure may have a gradient of composition from the core to the outside of the shell so that there is no precise boundary between core and shell but properties in centre of the core are different from properties on the outer boundary of shell.

In a configuration, nanoparticles are II-VI type semi-conductive nanoparticles and comprise a core based on cadmium, sulfur and selenium and are selected from:
- CdSe/CdS, CdSe/CdS/ZnS, CdSe/CdS/ZnSe, CdSe/CdS/ZnSe_{y}S_{(1-y)}, CdSe/ZnSe/ZnS, CdSe/ZnSeₓS₍₁₋ₓ₎/ZnS,
- CdSeₓS₍₁₋ₓ₎/ZnS, CdSeₓS₍₁₋ₓ₎/ZnSe, CdSeₓS₍₁₋ₓ₎/ZnSe_{y}S_{(1-y)}, CdSeₓTe₍₁₋ₓ₎/ZnS, CdSeₓTe₍₁₋ₓ₎/ZnSe,
- CdSe/Cd_{y}Zn_{(1-y)}S, CdSe/Cd_{y}Zn_{(1-y)}S/ZnS, CdSe/Cd_{y}Zn_{(1-y)}S/ZnSe, CdSe/Cd_{y}Zn_{(1-y)}S/ZnSe_{z}S_{(1-z)}
- CdSe/Cd_{y}Zn(_{1-y})Se, CdSe/Cd_{y}Zn_{(1-y)}Se/ZnS, CdSe/Cd_{y}Zn_{(1-y)}Se/ZnSe, CdSe/Cd_{y}Zn_{(1-y)}Se/ZnSe_{z}S_{(1-z)},
- CdSeₓS₍₁₋ₓ₎/CdS, CdSeₓS₍₁₋ₓ₎/CdS/ZnS, CdSeₓS₍₁₋ₓ₎/CdS/ZnSe, CdSeₓS₍₁₋ₓ₎/CdS/ZnSe_{y}S_{(1-y)},
- CdSeₓS₍₁₋ₓ)/Cd_{y}Zn_{(1-y)}S, CdSeₓS₍₁₋ₓ)/Cd_{y}Zn_{(1-y)}S/ZnS, CdSeₓS₍₁₋ₓ₎/Cd_{y}Zn_{(1-y)}S/ZnSe, CdSeₓS₍₁₋ₓ₎/Cd_{y}Zn_{(1-y)}S/ZnSe_{z}S_{(1-z)},
- CdSeₓS₍₁₋ₓ₎/Cd_{y}Zn_{(1-y)}Se, CdSeₓS₍₁₋ₓ)/Cd_{y}Zn_{(1-y)}Se/ZnS, CdSeₓS₍₁₋ₓ₎/Cd_{y}Zn_{(1-y)}Se/ZnSe, CdSeₓS₍₁₋ₓ₎/Cd_{y}Zn_{(1-y)}Se/ZnSe_{z}S_{(1-z)},
where x, y and z are rational numbers between 0 (excluded) and 1 (excluded), and emit light by fluorescence. Emitted light is typically a band centered on a wavelength in the visible range from 380 nm to 780 nm. Emitted light is typically a band having a FWHM less than 50 nm, preferably less than 30 nm, more preferably less than 20 nm.

In a configuration, nanoparticles are II-VI type semi-conductive nanoparticles and comprise a core based on zinc, sulfur and selenium and are selected from:
- ZnSe/ZnS, ZnSe/ZnSe_{y}S_{(1-y)}, ZnTe/ZnSe_{y}S_{(1-y)}
- ZnSeₓS₍₁₋ₓ₎/ZnS, ZnSeₓS₍₁₋ₓ₎/ZnSe, ZnSeₓS₍₁₋ₓ)/ZnSe_{y}S_{(1-y)}, ZnSeₓTe₍₁₋ₓ₎/ZnS, ZnSeₓTe₍₁₋ₓ₎/ZnSe, ZnSeₓTe₍₁₋ₓ₎/ZnSeₓS₍₁₋ₓ₎,
- ZnSe/Cd_{y}Zn_{(1-y)}S, ZnSe/Cd_{y}Zn_{(1-y)}S/ZnS, ZnSe/Cd_{y}Zn_{(1-y)}S/ZnSe, ZnSe/Cd_{y}Zn_{(1-y)}S/ZnSe_{z}S_{(1-z)}
- ZnSe/Cd_{y}Zn_{(1-y)}Se, ZnSe/Cd_{y}Zn_{(1-y)}Se/ZnS, ZnSe/Cd_{y}Zn_{(1-y)}Se/ZnSe, ZnSe/Cd_{y}Zn_{(1-y)}Se/ZnSe_{z}S_{(1-z)},
- ZnSeₓS₍₁₋ₓ₎/ZnS, ZnSeₓS₍₁₋ₓ₎/ZnS/ZnSe, ZnSeₓS₍₁₋ₓ₎/ZnS/ZnSe_{y}S_{(1-y)},
- ZnSeₓS₍₁₋ₓ₎/Cd_{y}Zn_{(1-y)}S, ZnSeₓS₍₁₋ₓ₎/Cd_{y}Zn_{(1-y)}S/ZnS, ZnSeₓS₍₁₋ₓ₎/Cd_{y}Zn_{(1-y)}S/ZnSe, ZnSeₓS₍₁₋ₓ₎/Cd_{y}Zn_{(1-y)}S/ZnSe_{z}S_{(1-z)},
- ZnSeₓS₍₁₋ₓ₎/Cd_{y}Zn_{(1-y)}Se, ZnSeₓS₍₁₋ₓ₎/Cd_{y}Zn_{(1-y)}Se/ZnS, ZnSeₓS₍₁₋ₓ₎/Cd_{y}Zn_{(1-y)}Se/ZnSe, ZnSeₓS₍₁₋ₓ₎/Cd_{y}Zn_{(1-y)}Se/ZnSe_{z}S_{(1-z)},
where x, y and z are rational numbers between 0 (excluded) and 1 (excluded), and emit light by fluorescence. Emitted light is typically a band centered on a wavelength in the visible range from 380 nm to 780 nm. Emitted light is typically a band having a FWHM less than 50 nm, preferably less than 30 nm, more preferably less than 20 nm.

In a configuration, nanoparticles are II-VI type semi-conductive nanoparticles and comprise a core based on zinc, cadmium, sulfur and selenium and are selected from:
- Cd_{w}Zn_{(1-w)}Se/CdS, Cd_{w}Zn_{(1-w)}Se/CdS/ZnS, Cd_{w}Zn_{(1-w)}Se/ZnSe/ZnS, Cd_{w}Zn_{(1-w)}Se/CdS/ZnSe, Cd_{w}Zn_{(1-w)}Se/CdS/ZnSe_{y}S_{(1-y)},
- Cd_{w}Zn_{(1-w)}SeₓS₍₁₋ₓ₎/ZnS, Cd_{w}Zn_{(1-w)}SeₓS₍₁₋ₓ₎/ZnSe, Cd_{w}Zn_{(1-w)}SeₓS₍₁₋ₓ₎/ZnSe_{y}S_{(1-y)}, Cd_{w}Zn_{(1-w)}SeₓTe₍₁₋ₓ₎/ZnS, Cd_{w}Zn_{(1-w)}SeₓTe₍₁₋ₓ₎/ZnSe,
- Cd_{w}Zn_{(1-w)}Se/Cd_{y}Zn_{(1-y)}S, Cd_{w}Zn_{(1-w)}Se/Cd_{y}Zn_{(1-y)}S/ZnS, Cd_{w}Zn_{(1-w)}Se/Cd_{y}Zn_{(1-y)}S/ZnSe, Cd_{w}Zn_{(1-w)}Se/Cd_{y}Zn_{(1-y)}S/ZnSe_{z}S_{(1-z)}
- Cd_{w}Zn_{(1-w)}Se/Cd_{y}Zn_{(1-y)}Se, Cd_{w}Zn_{(1-w)}Se/Cd_{y}Zn_{(1-y)}Se/ZnS, Cd_{w}Zn_{(1-w)}Se/Cd_{y}Zn_{(1-y)}Se/ZnSe, Cd_{w}Zn_{(1-w)}Se/Cd_{y}Zn_{(1-y)}Se/ZnSe_{z}S_{(1-z)},
- Cd_{w}Zn(_{1-w})SeₓS(₁₋ₓ₎/CdS, Cd_{w}Zn_{(1-w)}SeₓS₍₁₋ₓ₎/CdS/ZnS, Cd_{w}Zn_{(1-w)}SeₓS₍₁₋ₓ₎/CdS/ZnSe, Cd_{w}Zn_{(1-w)}SeₓS₍₁₋ₓ₎/CdS/ZnSe_{y}S_{(1-y)},
- Cd_{w}Zn_{(1-w)}SeₓS₍₁₋ₓ₎/Cd_{y}Zn_{(1-y)}S, Cd_{w}Zn_{(1-w)}SeₓS₍₁₋ₓ₎/Cd_{y}Zn_{(1-y)}S/ZnS, Cd_{w}Zn_{(1-w)}SeₓS₍₁₋ₓ₎/Cd_{y}Zn_{(1-y)}S/ZnSe, Cd_{w}Zn_{(1-w)}SeₓS₍₁₋ₓ₎/Cd_{y}Zn_{(1-y)}S/ZnSe_{z}S_{(1-z)},
- Cd_{w}Zn_{(1-w)}SeₓS₍₁₋ₓ₎/Cd_{y}Zn_{(1-y})Se, Cd_{w}Zn_{(1-w)}SeₓS₍₁₋ₓ₎/Cd_{y}Zn_{(1-y)}Se/ZnS, Cd_{w}Zn_{(1-w)}SeₓS₍₁₋ₓ₎/Cd_{y}Zn_{(1-y)}Se/ZnSe,
   Cd_{w}Zn_{(1-w)}SeₓS₍₁₋ₓ₎/Cd_{y}Zn_{(1-y)}Se/ZnSe_{z}S_{(1-z)},
where w, x, y and z are rational numbers between 0 (excluded) and 1 (excluded), and emit light by fluorescence. Emitted light is typically a band centered on a wavelength in the visible range from 380 nm to 780 nm. Emitted light is typically a band having a FWHM less than 50 nm, preferably less than 30 nm, more preferably less than 20 nm.

Most preferred II-VI nanoparticles are CdSe/CdS/ZnS, CdSeₓS₍₁₋ₓ₎/CdS/ZnS, CdSe/ZnSe/ZnS, CdSe/ZnSeₓS₍₁₋ₓ₎/ZnS, CdSeₓS₍₁₋ₓ₎/ZnSe/ZnS, CdₓZn₍₁₋ₓ₎Se/ZnSe/ZnS.

Other particularly suitable nanoparticles are III-V type semi-conductive nanoparticles and are selected from InP/ZnS, InP/ZnSe, InP/ZnSeₓS₍₁₋ₓ₎, InP/CdS/ZnS, InP/ZnSe/ZnS, InP/ZnSeₓS₍₁₋ₓ₎/ZnS, InP/GaP, CuₓIn_{y}Zn_{(1-x-y})S/ZnS, InₓAs₍₁₋ₓ₎P/ZnSeₓS₍₁₋ₓ₎, CsPbBr₃, CsPbI₃, FAPbI₃, where FA stands for formamidinium and x and y are rational numbers between 0 (excluded) and 1 (excluded).

Other particularly suitable nanoparticles are I-III-VI₂ type semi-conductive nanoparticles and are selected from AgInS₂, AgInSe₂, CuₓIn₍₁₋ₓ₎S₂, CuₓIn₍₁₋ₓ₎Se₂, especially CuInS₂ and CuInSe₂.

Other particularly suitable nanoparticles are selected from doped quantum dots as core, such as ZnSe:Mn/ZnS, or ZnSe:Cu/ZnS.

Other particularly suitable nanoplates are selected from ZnTe/ZnSe_{y}S_{(1-y)}, ZnSeₓTe₍₁₋ₓ₎/ZnS, ZnSeₓTe₍₁₋ₓ₎/ZnSe, ZnSeₓTe₍₁₋ₓ₎/ZnSe_{y}S_{(1-y)}, where x and y are rational numbers between 0 (excluded) and 1 (excluded).

### Matrix

In this disclosure, the matrix is a metal oxide compound comprising at least aluminum and zirconium as metallic elements, and having the formula (I):

AlₓZr_{y}M_{z}O (I)

In formula (I), M represents one or more metal elements selected from the group of Si, Ti, Hf, Ge, Sn, or mixtures thereof. Doping the matrix with titanium improves the electric conduction of the matrix - intrinsically isolating - and allows to hinder electric charge accumulation following optical excitation of quantum-dot - which yields a pair of charges in the dots. Concentration of titanium for doping is preferably less than 10 %, where the atomic percentage is given for metallic elements only. Doping the matrix with hafnium allows to increase the density of the matrix and/or adjust the refractive index of the matrix. These properties are advantageous in order to lower the refractive index difference between the matrix and the fluorescent nanoparticles. Doping the matrix with silicon leads to lower permeability to water, thereby increasing the protection efficiency of the matrix against degradation of fluorescent nanoparticles in humid conditions. More generally, dopant can be adapted to adjust the refractive index or the conductivity of the matrix.

Also, in the present disclosure, the composition of the matrix is theorical, and the atomic ratios should be understood as an aimed composition, provided that the final composition may comprise up to 10at% of impurities. The impurities are additional elements that may be included in the composition, which are not aluminium, zirconium, or a selected dopant. These impurities may be organic moieties coming from precursors of the metal oxide matrix. A matrix comprising less than 10 wt% of impurities is considered pure.

In formula (I), the stoichiometric coefficient of oxygen is fixed to 1. Therefore, parameters x, y and z are linked, depending on the respective coordination numbers of Al, Zr and M elements in the metal oxide matrix.

In addition, x is in the range from 0 to 2/3; y is in the range from 0 to 1/2; and z is in the range from 0 to 2/5. When z is null, then the relation between x and y is (3/2x+2y)=1, so as to respect coordination numbers of alumina and zirconia.

In a preferred embodiment, the matrix is zirconium oxide - ZrO₂, x=0, z=0.

In a preferred embodiment, x is in the range from 1/40 to 8/15; y is in the range from 1/10 to 77/160. More preferably x is in the range from 1/20 to 8/15; y is in the range from 1/10 to 37/80. Even more preferably, x is in the range from 1/10 to 8/15; y is in the range from 1/10 to 17/40. The following table I shows the equivalence between values of x and y - z being null - in terms of atomic percentage of Aluminum and Zirconium.

**Table I**

| Al - x | Zr - y | 1,5x+2y | Al at% | Zr at% |
|---|---|---|---|---|
| 0 | 1/2=0.5 | 1 | 0,0% | 100,0% |
| 1/80=0.0125 | 157/320=0.490625 | 1 | 2,5% | 97,5% |
| 1/40=0.025 | 77/160=0.48125 | 1 | 4,9% | 95,1% |
| 1/20=0.05 | 37/80=0.4625 | 1 | 9,8% | 90,2% |
| 1/10=0.1 | 17/40=0.425 | 1 | 19,0% | 81,0% |
| 8/15 | 1/10=0.01 | 1 | 84,2% | 15,8% |
| 3/5=0.6 | 1/20=0.05 | 1 | 92,3% | 7,7% |
| 3/7 | 5/28 | 1 | 70,6% | 29,4% |

For instance, the metal oxide matrix corresponding to x=2/9, y=1/3 and z=0 is Al_{2/9}Zr_{1/3}O, comprising 40 at% of aluminium and 60 at% of zirconium, where the atomic percentage is given for metallic elements only.

Similarly, the metal oxide matrix corresponding to x=8/15, y=1/10 and z=0 is Al_{1/15}Zr_{1/10}O, comprising 84 at% of aluminium and 16 at% of zirconium, where the atomic percentage is given for metallic elements only.

Similarly, the metal oxide matrix corresponding to x=6/17, y=4/17 and z=0 is Al_{6/17}Zr_{4/17}O, comprising 60 at% of aluminium and 40 at% of zirconium, where the atomic percentage is given for metallic elements only. Compositions of metal oxide matrix with x=6/17±0.03 and y=4/17±0.03 are especially efficient to protect fluorescent nanoparticles.

Similarly, the metal oxide matrix corresponding to x=2/5, y=1/5 and z=0 is Al_{2/5}Zn_{1/5}O, comprising 66,7 at% of aluminium and 33.3 at% of zirconium, where the atomic percentage is given for metallic elements only. Compositions of metal oxide matrix with x=2/5±0.03 and y=1/5±0.03 are especially efficient to protect fluorescent nanoparticles.

Similarly, the metal oxide matrix corresponding to x=3/7; y=5/28 and z=0 is Al_{3/7}Zr_{5/28}O, comprising 70 at% of aluminium and 30 at% of zirconium, where the atomic percentage is given for metallic elements only.

Similarly, a metal oxide matrix corresponding to x=1/10 and y=1/10 may have a value for z varying from z=13/40 if all elements represented by M have a coordination number of 2 - MO₂ - to z=13/30 if all elements represented by M have a coordination number of 1.5 - M₂O₃.

In a preferred embodiment, x is in the range from 2/9 to 6/17.

In a preferred embodiment, y is in the range from 4/17 to 1/3.

In an embodiment, aluminum oxide and zirconium oxide represent more than 90 wt% of the matrix, preferably more than 95 wt% of the matrix.

In an embodiment, the matrix further comprises a concentration ranging from 0.1 at% to 15 at% of metal elements selected from silicon (Si), titanium (Ti), hafnium (Hf), germanium (Ge), or tin (Sn) where the atomic percentage is given for metallic elements only.

### Composite particles

In this disclosure, fluorescent nanoparticles are encapsulated in a metal oxide matrix so as to form composite particles.

In an embodiment, the loading charge of the fluorescent nanoparticles in the composite particle is at least 1%, preferably at least 2.5%, more preferably at least 5%, said loading charge being the mass ratio between the mass of fluorescent nanoparticles comprised in a composite particle and the mass of said composite particle. Indeed, the performance of composite particles is proportional to the concentration of fluorescent nanoparticles they contain. Therefore, a high concentration of fluorescent nanoparticles is advantageous. It has to be noted however that increasing concentration of fluorescent nanoparticles without degrading their properties - as a consequence of aggregation or manufacturing process for instance - is not easy.

More precisely, for nanoparticles emitting green fluorescent light - between 500 nm and 580 nm - when irradiated with blue light with a wavelength of 450 nm, the loading charge is preferably at least 2.5%, more preferably at least 5%, optionally at least 10%. For nanoparticles emitting red fluorescent light - between 600 nm and 680 nm - when irradiated with blue light with a wavelength of 450 nm, the loading charge is preferably at least 1%, more preferably at least 2%, optionally at least 5%.

In some embodiments, the loading charge of the fluorescent nanoparticles in the composite particle is even lower than 1%. This is especially desirable with fluorescent nanoparticles having a strong absorption and PLQY: a low concentration of fluorescent nanoparticles is sufficient to achieve the light conversion expected. A loading charge of the fluorescent nanoparticles in the composite particle in the range from 0.1% to 1% may be preferred. This is especially relevant for red fluorescent nanoparticles.

In addition, the geometrical distribution of fluorescent nanoparticles in composite particles may be controlled in several ways.

In an embodiment, more than 95 wt% of the fluorescent nanoparticles in a composite particle are dispersed in the inner part of the composite particle. By inner part, it is meant the following: the composite particle consists of an inner part surrounded by an external layer. The external layer covers the whole inner part. The external layer represents less than 5 wt% of the composite particle, and the external layer has a thickness greater than or equal to 5 nm. In a preferred embodiment, the thickness of the external layer is greater than or equal to 15 nm.

In an embodiment, the fluorescent nanoparticles are uniformly dispersed in the matrix. By uniformly, it is meant that fluorescent nanoparticles are not aggregated, are not in contact, and are separated by metal oxide matrix. Each fluorescent nanoparticle is spaced from adjacent fluorescent nanoparticles by an average minimal distance.

In an embodiment, more than 95 wt% of the fluorescent nanoparticles in a composite particle are uniformly dispersed in the inner part of the composite particle.

In an embodiment, at least two different type of fluorescent nanoparticles are dispersed in the matrix. For instance, a first type of nanoparticles emitting green light by fluorescence and a second type of nanoparticles emitting red light by fluorescence are mixed in the same composite particles. When illuminated with a single excitation light - typically blue light with a wavelength around 450 nm - green and red light may be emitted simultaneously by such composite particles. In a preferred embodiment, the ratio of weight concentration of green fluorescent nanoparticles divided by weight concentration of red fluorescent nanoparticles is in the range from 25 to 4, preferably in the range from 20 to 5. For clarity, a ratio of weight concentration of green fluorescent nanoparticles divided by weight concentration of red fluorescent nanoparticles equal to ten correspond to composite particles comprising ten times more - in weight - green fluorescent nanoparticles than red fluorescent nanoparticles.

Accordingly, it is possible to provide a test comprising a plurality of bio-marked composite particles wherein each bio-marked composite particle allows identification of a specific antibody, and each bio-marked composite particle has a distinguishing fluorescence signature determined by its fluorescent nanoparticles' combination. Therefore, such a test is able testing multiple antibodies in a single test.

In an embodiment, the composite particles present a low specific surface area - comprising two contributions of surface roughness and internal porosity - which can be characterized by adsorption-desorption of nitrogen in the Brunauer-Emmett-Teller (BET) theory. The low specific surface area is associated to a difficult penetration of oxygen or water through the matrix. Indeed, the matrix presents high barrier properties to protect the fluorescent nanoparticles from degradation. Low BET values, lower than 15 m²/g at a nitrogen pressure of 650 mmHg, preferably lower than 5 m²/g at a nitrogen pressure of 650 mmHg are preferred. However, the composite particles may present a higher BET value, up to 50 m²/g and be still highly protective against oxygen or water degradation. Without being bound by theory, it seems that the matrix may have a large macroscopic porosity, but a very dense matrix around the fluorescent nanoparticles, leading to a large BET value. In addition, the external layer devoid of fluorescent nanoparticles may be porous without affecting the overall barrier properties of the matrix.

The composite particles may be in the form of a monodisperse population. Monodisperse composite particles are advantageous for various reasons, depending on the domain of application. When composite particles are used in fluid process, such as cytometry flow assays in medical diagnostic, a homogeneous size distribution leads to more reproductible results.

The mean size of the composite particles is preferably in a range from 50 nm to 50 µm, more preferably from 100 nm to 10 µm, even more preferably from 200 nm to 5 µm. Composite particles having a mean size from 500 nm to 2000 nm are especially suitable for assays, where the size of one composite particle governs the light intensity available by fluorescence for identification of a positive association between a target analyte and said composite particle.

All the technically acceptable combinations AᵢBⱼ of the following structures - as fluorescent nanoparticles as such, or as heterostructured nanoparticles comprising said structure as a core - and matrix yield suitable composite particles :
- Nanoparticles:
   A1. Phosphide III-V quantum dots selected from the group of InP, Cd₃P₂, Zn₃P₂, AlP, GaP, TlP;
   A2. II-VI quantum dots selected from the group of CdSe, CdSeS, ZnSe, ZnSeS;
   A3. ternary I-III-VI₂ quantum dots selected from the group of CuInS₂, CuInSe₂, AgInS₂, AgInSe₂;
   A4. quantum dots selected from the group of CuInZnS, CuInZnSe.
- Matrix:
   B1. Matrix of formula AlₓZr_{y}O with x in the range from 2/9 to 6/17 and y in the range from 4/17 to 1/3. Matrix composed solely of alumina and zirconia - *i.e.,* with z=0 - are especially dense, thereby limiting gas or harmful chemicals diffusion into the composite particles.
   B2. Matrix of formula AlₓZr_{y}M_{z}O with M representing silicon element; x in the range from 1/20 to 8/15; y in the range from 1/10 to 37/80; and z in the range from 1/100 to 1/20. Addition of silicon in the matrix leads to lower permeability to water.
   B3. Matrix of formula AlₓZr_{y}M_{z}O with M representing titanium element; x in the range from 1/20 to 8/15; y in the range from 1/10 to 37/80; and z in the range from 1/100 to 1/20. Addition of titanium allows to prevent electric charge accumulation following optical excitation of quantum-dot.
   B4. Matrix of formula AlₓZr_{y}M_{z}O with M representing hafnium element; x in the range from 1/20 to 8/15; y in the range from 1/10 to 37/80; and z in the range from 1/100 to 1/20. Addition of hafnium allows to increase the density of the matrix and/or adjust the refractive index of the matrix.

Hereby, a list of composite particles that may be obtained : CdSe/ZnSe/ZnS in a matrix Zr-100% ; CdSe/ZnSe/ZnS in a matrix Al/Zr-(67/33) ; CdSe/CdS/ZnS in a matrix Zr-100% ;CdSe/CdS/ZnS in a matrix Al/Zr-(67/33) ; InP/ZnSe/ZnS in a matrix Zr-100% ; InP/ZnSe/ZnS in a matrix Al/Zr-(67/33) ; CuInZnS/ZnS in a matrix Zr-100% ; CuInZnS/ZnS in a matrix Al/Zr-(67/33) ; CuInZnSe/ZnSe/ZnS in a matrix Zr-100% ; CuInZnSe/ZnSe/ZnS in a matrix Al/Zr-(67/33) - all percentages of aluminum and zirconium are atomic percentages.

Composite particles have a diameter that is greater than 100 nm, preferably greater than 700 nm and even more preferably greater than 1µm.

### Biomolecules

Biomolecules 200 are attached to the external surface of the metal oxide matrix of the composite particle. Biomolecules 200 comprises an anchor. Said anchor comprises histidine residues and preferably comprises at least 4 consecutive histidine residues, and more preferably at least 6 consecutive histidine residues. In other words, the anchor is configured to bind with the metal oxide matrix of the composite particle. The anchor may be present within the biomolecule 200 naturally; or may be artificially grafted.

Biomolecules 200 may be proteins, nucleic acids, hormones, antigens, antibody, viruses' parts or a mix thereof. For instance, biomolecules 200 may be Sars-COV2, Diphtheria (toxin/anatoxin), Tetanus (toxin/anatoxin), Poliomyelitis (inactivated/ lysed/ or fragments), Pertussis : Bordetella pertussis (anatoxin/fragments/recombinant proteins), Haemophilus B (fragments from Haemophilus influenzae B), Hepatitis B (virus surface antigens), Pneumococcus : (fragments from Streptoccocus pneumoniae), Meningococcus C (fragments from Neisseria meningitidis C), Measles (inactivated or lysed, VLP, fragment or recombinant protein), Mumps (inactivated or lysed, VLP, fragment or recombinant protein), Rubella (inactivated or lysed, VLP, fragment or recombinant protein), Influenza (recombinant surface antigen (HA and NA)), Yellow fever (inactivated or lysed, VLP, fragment or recombinant protein), Meningococcal B (fragments from Neisseria meningitidis (B), recombinant surface antigen), other natural allergens including, but not limited to, peanuts (Ara h 2, Ara h 3, Ara h 6, Ara h 9), Hazelnut (Cor a 1, Cor a 8, Cor a 14), pollen (Phl p 1, Phl p 6, Phl p 12, Bet v 1, ), dust mite (Der p 1, Der p 2), Egg white (Gal d 1), bovine milk (Bos d 4), felines (Fel d 1), apple (Mal d 1), peach (Pru p 1), and/or plant rot fungus (Alt A 1)

### Method of preparation

The invention also relates to a method of preparation of a composite particle as disclosed hereabove, wherein the metal oxide matrix is obtained by hydrolysis of precursors, the precursors being selected for example in the group of aluminium oxide, titanium oxide, zirconium oxide or hafnium oxide may be selected from the group of carboxylates, carbonates, thiolates, alkoxides, oxides, sulfates, phosphates, nitrates, acetates, chlorides, bromides, acetylacetonate or a mixture thereof; preferably the precursors are alkoxides.

The composite particles may be obtained by the method disclosed in WO2018/220165, the full content of which is included by reference, in which the precursors of the metal oxide matrix are aluminium alkoxides, silicon oxide, zirconium alkoxides and optionally other metal alkoxides.

Metal alkoxides are largely available compounds, with good compatibility between them, allowing to prepare mixture of alkoxides leading by condensation - in acidic or basic conditions - to metal oxide having a mixed composition, as disclosed hereabove. The molar ratio of metal alkoxides defines the atomic percentage of metal in the metal oxide obtained after condensation.

Preferred aluminum alkoxides are aluminum tert-butoxyde.

Preferred zirconium alkoxides are zirconium tetra propoxide Zr(OC₃H₇)₄, for instance as a solution in 1-propanol.

Preferred silicon alkoxides are tetramethoxysilane Si(OCH₃)₄ (commonly called methyl silicate) and tetraethoxysilane Si(OC₂Hₛ)₄ (commonly called ethyl silicate), methyltriethoxysilane, or a mixture thereof.

Preferred titanium alkoxides are titanium tetraisopropoxide Ti(OisoC₃H₇)₄., titanium tetraethoxide or titanium tetrabutoxide, or a mixture thereof.

Preferred hafnium alkoxides are hafnium methoxide, hafnium n-propoxide, hafnium isopropoxide, or hafnium ethoxide, or a mixture thereof.

The matrix of the composite particle may be obtained by nebulization of a composition in a heated enclosure, said composition comprising fluorescent nanoparticles; and precursors of a matrix. The precursor may be a precursor of aluminium oxide, titanium oxide, zirconium oxide, hafnium oxide, silicon oxide or mixtures thereof.

Composite particles are mixed with biomolecules to obtain bio-marked composite particles 10. More concretely, the composite particles and the biomolecules are mixed in a buffer. Preferably, the mixing lasts more than 10 minutes, preferably more than 1 hour. The buffer may be, for instance, water or physiological serum.

Optionally, prior to the mixing with the composite particles, biomolecules may be grafted with an anchor. The grafted anchor comprises at least four histidine residues. More preferably, the grafted anchor comprises at least four consecutive histidine residues, and even more preferably at least six consecutive histidine residues.

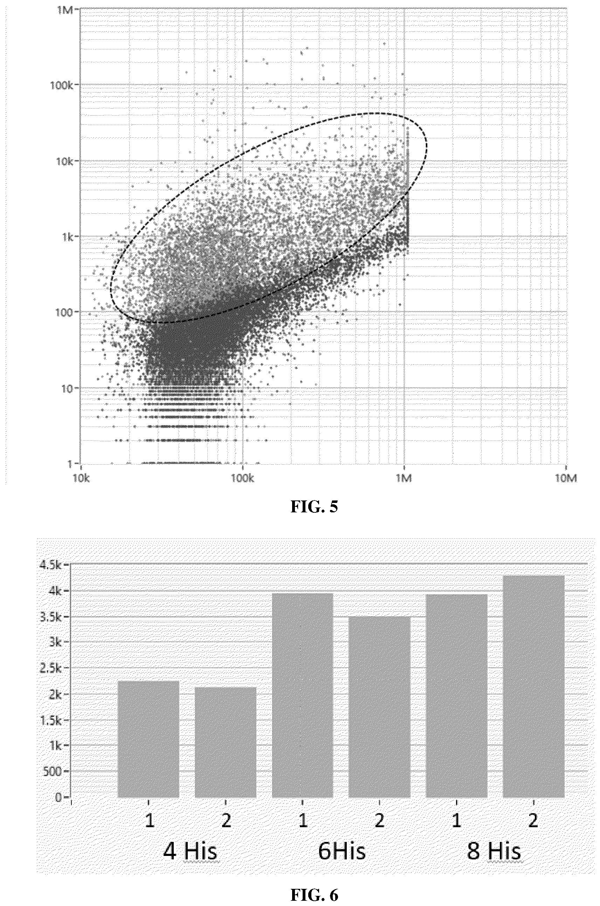

Figure 6 represents a histogram representing, for a population of bio-marked composite particles, the mean fluorescence intensity at the emission wavelength of the fluorescent probe as a function of the number of consecutive histidine residues included in the anchor of the biomolecule of the bio-marked composite particles. The mean fluorescent intensity is obtained by analysing the population of bio-marked composite particles in a cytometry process. It is noted that each measurement is reproduced two times (samples 1 and 2), by analysing two populations of bio-marked composite particles having a definite number of consecutive histidine residues,

It is observed that the mean fluorescence is higher when the anchor comprises six consecutive histidine residues, compared to when the anchor comprises four consecutive histidine residues. However, the mean fluorescence when the anchor comprises six consecutive histidine residues is comparable to the mean fluorescence when the anchor comprises height consecutive histidine residues.

This result shows that the attachment of the biomolecule to the composite particle is stronger when the anchor comprises at least six consecutive histidine residues.

### EXAMPLES

The present invention is further illustrated by the following examples.

### Method for testing antibodies

For each of these examples, the bio-marked composite particle has been brought into contact with a solution comprising antibodies, wherein at least one of the antibodies of the solution is able to bound with the biomolecule. Then, the bio-marked composite particle coupled with antibodies have been treated with a fluorescent probe 303.

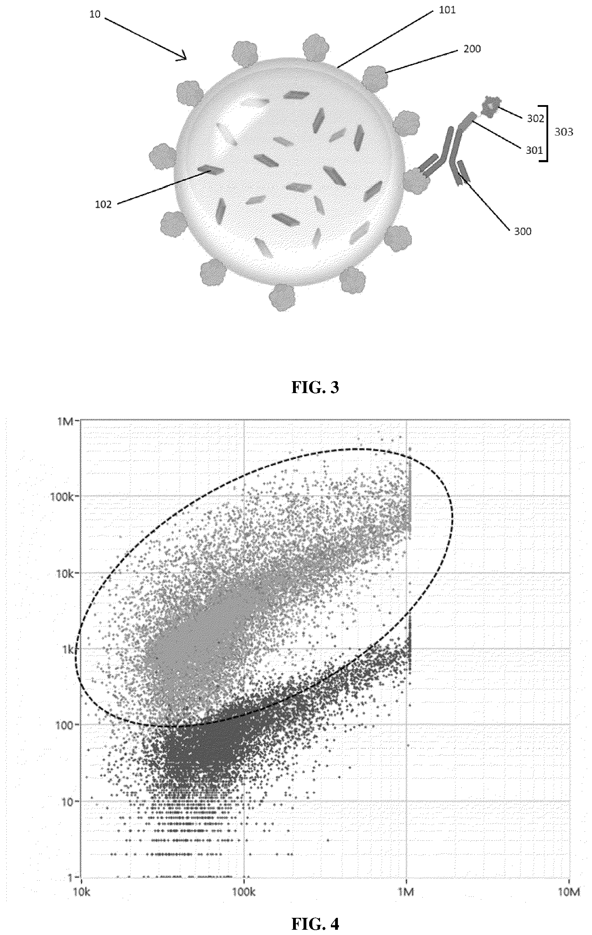

Figure 3 is a schema illustrating a bio-marked composite particle 10 according to the invention attached to an antibody 300 and a fluorescent probe 303. The fluorescent probe 303 comprises a coupling part 301 and a fluorescent part 302. The coupling part 301 is configured to selectively bound with a specific predetermined antibody. The fluorescent part 302 is configured to provide a fluorescence signal when excited by a specific excitation. This excitation may be a light excitation. Advantageously, the wavelength of the light excitation of the fluorescent part 302 is the same as the wavelength of the light excitation of the fluorescent nanoparticles of the bio-marked composite particle.

In the following examples, the coupling part 301 is a protein of interest that can be a reconstruction of a pathogen or allergen target including: Sars-COV2, Diphtheria (toxin/anatoxin), Tetanus (toxin/anatoxin), Poliomyelitis (inactivated/ lysed/ or fragments), Pertussis : Bordetella pertussis (anatoxin/fragments/recombinant proteins), Haemophilus B (fragments from Haemophilus influenzae B), Hepatitis B (virus surface antigens), Pneumococcus : (fragments from Streptoccocus pneumoniae), Meningococcus C (fragments from Neisseria meningitidis C), Measles (inactivated or lysed, VLP, fragment or recombinant protein), Mumps (inactivated or lysed, VLP, fragment or recombinant protein), Rubella (inactivated or lysed, VLP, fragment or recombinant protein), Influenza (recombinant surface antigen (HA and NA)), Yellow fever (inactivated or lysed, VLP, fragment or recombinant protein), Meningococcal B (fragments from Neisseria meningitidis (B), recombinant surface antigen), other natural allergens including, but not limited to, peanuts (Ara h 2, Ara h 3, Ara h 6, Ara h 9), Hazelnut (Cor a 1, Cor a 8, Cor a 14), pollen (Phl p 1, Phl p 6, Phl p 12, Bet v 1, ), dust mite (Der p 1, Der p 2), Egg white (Gal d 1), bovine milk (Bos d 4), felines (Fel d 1), apple (Mal d 1), peach (Pru p 1), and/or plant rot fungus (Alt A 1) and the fluorescent part 302 is the mGreenLantern^{©} (mGL) protein. The fluorescent part 302 may also be other organic or inorganic fluorophores including, but not limited to, mTurquoise, mCerulean, mGFP (Green Fluorescent Protein), mCherry, Cyanine (Cy2, Cy 3, Cy 3.5, Cy5.5), Alexa type (Alexa 488, Alexa 555, Alexa 647), Atto type (Atto 488, Atto 550, Atto 655, Atto 647N), other Quantum Dots (such as, but not limited to CdSe, ZnS, ZnSe) which emit at a different wavelength compared to the material encapsulated within the matrix material.

The probed bio-marked composite particles 10 are then tested through a flow cytometry method.

### Example 1

CdSe nanoparticles were prepared according to methods of the art (Lhuillier E. et al., Acc. Chem. Res., 2015, 48 (1), pp 22-30; Pedetti S. et al., J. Am. Chem. Soc., 2014, 136 (46), pp 16430-16438; Ithurria S. et al., J. Am. Chem. Soc., 2008,130,16504-16505; Nasilowski M. et al., Chem. Rev. 2016, 116, 10934-10982).

The CdSe nanoparticles were treated by the method disclosed in WO2018/220165 with aluminum tert-butoxyde and zirconium tetra propoxide precursors leading to a matrix of composition A1_{6/17}Zr_{4/17}O, comprising 60 at% of aluminium and 40 at% of zirconium, in which CdSe nanoparticles are embedded. The loading charge of CdSe nanoparticles in the composite particles is set to 0.5%.

The obtained composite particle has been mixed with SARS-CoV2 proteins in water. SARS-CoV2 comprises residues presenting at least four consecutive histidine residues. The resulting dispersion has been mixed in water for two hours. This dispersion is a dispersion of bio-marked composite particles.

### Other examples

Example 1 is reproduced with the composite particles defined in the following

**table**

| Nanoparticles | Matrix | | | Loading charge | Probe | Biomolecule |
|---|---|---|---|---|---|---|
| | x | y | z (M) | | | |
| CdSe dots - 5 nm (sample A1) | 6/17 | 4/17 | 0 | 0.5% | mGL | Sars-Cov2 |
| CdSe dots - 5 nm (sample A2) | 6/17 | 4/17 | 0 | 0.5% | mGL | Neisseria meningiditus |
| ZnSe dots - 5 nm | 2/9 | 1/3 | 0 | 5% | mCherry | Neisseria meningiditus |
| Core/shell/shell nanoplatelets A | 6/17 | 4/17 | | 1.5% | mCerulan | Ara h 2 |
| Core/shell/shell nanoplatelets B | 1/20 | 9/20 | 0.017 (Hf) | 2% | mGL | Pneumococcus |
| Core/shell/shell nanospheres C | 6/17 | 4/17 | 0 | 1% | mGFP | Bordetella pertussis |
| Core/shell/shell nanospheres D | 2/5 | 115 | 0 | 15% | mCherry | Yellow fever |
| Core/shell/shell nanoplatelets E | 6/17 | 4/17 | 0 | 3% | mGL | Fel d 1 |
| Core/shell/shell nanospheres F | 2/5 | 115 | 0 | 1% | mTurquoise | HIV |
| Core/shell nanospheres G | 6/17 | 4/17 | 0 | 2% | mGL | Phl p 1 |
| Core/shell nanospheres H | 1/10 | 17/40 | 0 | 15% | Cy3.5 | Tetanus |
| Core/shell/shell nanoparticles I | 8/15 | 1/10 | 0 | 15% | Alexa 488 | Rubella |
| Core/shell/shell nanoplatelets J | 1/40 | 77/60 | 0 | 10% | mGFP | Maid 1 |
| Core/shell nanospheres K | 1/20 | 9/20 | 0.017 (Si) | 15% | mCherry | Diphtheria |
| Core/shell nanoparticles L | 2/9 | 1/3 | 0 | 15% | Cy2 | Alt A 1 |
| Core/shell nanoparticles M | 2/5 | 115 | 0 | 12% | mCerulan | Cor a 8 |
| Core/shell/shell nanoparticles N | 1/20 | 9/20 | 0.017 (Ti) | 15% | mTurquoise | Poliomyelitis |
| Core/shell/shell nanoparticles O | 1/40 | 9/20 | 0.031 (Ti) | 15% | mCerulan | Meningococcal B |
| Mixture of Core/shell/shell nanoplatelets A (1.5%) and J (10%) | 1/10 | 17/40 | 0 | 11.5% | Atto 550 | Gal d 1 |
| Mixture of Core/shell/shell nanospheres F (1%) and Core/shell nanoparticles M (12%) | 8/15 | 1/10 | 0 | 13% | mTurquoise | Bos d 4 |

Core/shell/shell nanoplatelets A: CdSe_{0.45}S_{0.55}/Cd_{0.30}Zn_{0.70}S/ZnS, with a core of thickness 1.2 nm and a lateral dimension, *i.e*., length or width, greater than 8 nm and shells of thicknesses 2.5 nm and 2 nm, emitting red fluorescent light.

Core/shell/shell nanoplatelets B: CdSe_{0.72}S_{0.28}/CdS/ZnS, with a core of thickness 1.2 nm and a lateral dimension, *i.e*., length or width, greater than 8 nm and shells of thicknesses 3.5 nm and 0.5 nm, emitting red fluorescent light.

Core/shell/shell nanospheres C: CdSe/CdS/ZnS, with a core of diameter 4.5 nm and shell thicknesses of 1.5 nm and 1 nm, emitting red fluorescent light.

Core/shell/shell nanospheres D: CdSe/ZnSe/ZnS, with a core of diameter 4.5 nm and shell thicknesses of 1.5 nm and 1 nm, emitting green fluorescent light.

Core/shell/shell nanoplatelets E: CdSe_{0.72}S_{0.28}/ZnSe/ZnS, with a core of thickness 1.2 nm and a lateral dimension, *i.e*., length or width, greater than 8 nm and shells of thicknesses 3.5 nm and 0.5 nm, emitting red fluorescent light.

Core/shell/shell nanoparticles F: InP/ZnSe/ZnS with a core of diameter 4.0 nm and shell thicknesses of 2 nm and 1 nm, emitting red fluorescent light.

Core/shell/shell nanoparticles G: InP/ZnSe/ZnS with a core of diameter 3.0 nm and shell thicknesses of 2 nm and 1 nm, emitting red fluorescent light.

Core/shell/shell nanoparticles H: InP/ZnSe/ZnS with a core of diameter 2.5 nm and shell thicknesses of 1.5 nm and 1.25 nm, emitting green fluorescent light.

Core/shell/shell nanoparticles I: InP/ZnSe/ZnS with a core of diameter 2.5 nm and shell thicknesses of 2.75 nm and 2.50 nm, emitting green fluorescent light.

Core/shell/shell nanoplatelets J: CdSe_{0.10}S_{0.90}/ZnS/Cd_{0.20}Zn_{0.80}S, with a core of thickness 1.5 nm and a lateral dimension, *i.e.,* length or width, greater than 10 nm and shells of thicknesses 1 nm and 2.5 nm, emitting green fluorescent light.

Core/shell nanospheres K: CdSe_{0.10}S_{0.90}/ZnS, with a core of diameter 4 nm and shell thickness of 1 nm, emitting green fluorescent light.

Core/shell nanoparticles L: InP/ZnS, having a diameter of 3.5 nm, emitting green fluorescent light.

Core/shell nanoparticles M: InP/ZnSe, having a diameter of 4.4 nm, emitting green fluorescent light.

Core/shell/shell nanoparticles N: InP/GaP/ZnS, having a diameter of 7.2 nm, emitting green fluorescent light.

Core/shell/shell nanoparticles O: InP/ZnSe/ZnS, having a diameter of 7.4 nm, emitting green fluorescent light.

Figure 4 is a graphic representing the intensity of fluorescence for a population of bio-marked composite particles (sample A1). X axis represents the fluorescence intensity at the emission wavelength of the fluorescent nanoparticles while Y axis represents the fluorescence intensity at the emission wavelength of the fluorescent probe. The test is a flow cytometry test, for a sample comprising Sars-Cov2 bio-marked composite particles and a control sample.

The circled point cloud corresponds to the point cloud of the sample particles. It is observed that these particles show a florescence corresponding to the fluorescent probe and a fluorescence corresponding to the fluorescent nanoparticles. Thus, it shows that antibodies have effectively bounded to the bioparticles of the bio-marked composite particles.

On the contrary, the other point cloud, corresponding to the control, only shows a marginal fluorescence corresponding to the probe but shows a strong fluorescence corresponding to the semi-conductor nanoparticles. Thus, the control has not bound to any antibody.

Figure 5 is a graphic representing the intensity of fluorescence for a population of bio-marked composite particles (sample A2). X axis represents the fluorescence intensity at the emission wavelength of the fluorescent nanoparticles while Y axis represents the fluorescence intensity at the emission wavelength of the fluorescent probe. The test is a flow cytometry test, for a sample comprising Neisseria meningitidis bio-marked composite particles and a control sample.

The circled point cloud corresponds to the point cloud of the sample particles. It is observed that these particles show a florescence corresponding to the fluorescent probe and a fluorescence corresponding to the fluorescent nanoparticles. Thus, it shows that antibodies have effectively bounded to the bioparticles of the bio-marked composite particles.

On the contrary, the other point cloud, corresponding to the control, only shows a marginal fluorescence corresponding to the probe but shows a strong fluorescence corresponding to the semi-conductor nanoparticles. Thus, the control has not bound to any antibody.

## Claims

1. A bio-marked composite particle (10) comprising
• a plurality of fluorescent nanoparticles (102) encapsulated in a matrix (101) comprising aluminium oxide, titanium oxide, zirconium oxide, hafnium oxide, siliocn oxide or mixtures thereof; forming a composite particle and
• a plurality of biomolecules (200) anchored at the surface of the matrix (101) of the composite particle (10), said biomolecules (200) comprising an anchor, the anchor comprising at least four histidine residues.

2. The bio-marked composite particle (10) according to claim **1,** wherein the composition of the matrix (101) is
AlₓZr_{y}O (I) ;
wherein x, and y respectively represent the stoichiometric ratios of Aluminum and Zirconium;
x is in the range from 0 to 2/3;
y is in the range from 0 to 1/2; and
(3/2x+2y)=1.

3. The bio-marked composite particle (10) according to claim **1** or **2,** wherein the composition of the matrix (101) is pure ZrO₂.

4. The bio-marked composite particle (10) according to claim **1,** wherein the matrix (101) is a metal oxide compound of formula AlₓZr_{y}M_{z}O wherein
• x is in the range from 0 to 2/3;
• y is in the range from 0 to 1/2;
• M represents one or more metal elements selected from the group of Si, Hf, Ge, Sn, or mixtures thereof, and
• z is in the range from 0 to 2/5, 0 excluded.

5. The bio-marked composite particle (10) according to claim **1** to **4,** wherein the anchor comprises at least four consecutive histidine residues.

6. The bio-marked composite particle (10) according to any one of claims **1** to **5,** wherein the matrix (101) is obtained by nebulization of a composition in a heated enclosure, said composition comprising
• fluorescent nanoparticles (102); and
• precursors of aluminium oxide, titanium oxide, zirconium oxide, hafnium oxide, silicon oxide or mixtures thereof.

7. The bio-marked composite particle (10) according to claim 5, wherein the precursors of aluminium oxide, titanium oxide, zirconium oxide, hafnium oxide or silicon oxide are selected from the group of carboxylates, carbonates, thiolates, alkoxides, oxides, sulfates, phosphates, nitrates, acetates, chlorides, bromides, acetylacetonate or a mixture thereof; preferably the precursors are alkoxides.

8. The bio-marked composite particle (10) according to any one of claims **1** to **7,** wherein the fluorescent nanoparticles (102) are quantum dots.

9. The bio-marked composite particle (10) according to any one of claims **1** to **7,** wherein the fluorescent nanoparticles (102) are quantum nanoplates.

10. The bio-marked composite particle (10) according to any one of claims **1** to **9,** wherein the biomolecules (200) are selected from the group consisting of proteins, nucleic acids, hormones, antigens, antibody, viruses' parts or a mix thereof.

11. Method for synthetizing a bio-marked composite particle (10) comprising:
• Providing composite particles comprising a plurality of fluorescent nanoparticles (102) encapsulated in a matrix (101) comprising aluminium oxide, titanium oxide, zirconium oxide, hafnium oxide, silicon oxide or mixtures thereof;
• Providing biomolecules (200) comprising an anchor, the anchor comprising at least four histidine residues;
• Mixing the composite particles and the biomolecules (200) in a buffer.

12. Method according to claim **11,** comprising, prior to providing biomolecules comprising an anchor, providing biomolecules and grafting an anchor to said biomolecules, the anchor comprising at least four histidine residues.

13. Method for detecting antibodies for a biomolecule in a sample, the method comprising:
• Sampling a fluid sample of a patient;
• Adding the sample to a dispersion comprising the bio-marked composite particles (10) of any of claims **1** to **10** comprising said biomolecule, to obtain a test sample;
• Adding a fluorescent probe to the test sample to obtain a probed test sample;
• Analyzing the probed test sample through a flow cytometry process.
